(19) Europäisches Patentamt
European Patent Office
Office européen des brevets

(11) Numéro de publication: **0 376 849 A1**

(12) # DEMANDE DE BREVET EUROPEEN

(21) Numéro de dépôt: **89403668.0**

(22) Date de dépôt: **28.12.89**

(51) Int. Cl.5: **C07D 243/12, C07D 403/12, C07K 5/06, A61K 31/55, A61K 37/02**

(30) Priorité: **29.12.88 FR 8817395**

(43) Date de publication de la demande: **04.07.90 Bulletin 90/27**

(84) Etats contractants désignés: **CH DE GB IT LI NL**

(71) Demandeur: **ROUSSEL-UCLAF**
**35, boulevard des Invalides**
**F-75007 Paris(FR)**

(72) Inventeur: **Gasc, Jean-Claude**
**6, rue Georges Lyssandre**
**F-93140 Bondy(FR)**
Inventeur: **Humbert, Daniel**
**15, rue Gaston Charle**
**F-94120 Fontenay Sous Bois(FR)**

(74) Mandataire: **Vieillefosse, Jean-Claude et al**
**Roussel-Uclaf 111, route de Noisy B.P. 9**
**F-93230 Romainville(FR)**

(54) **Nouveaux dérivés de la 2,4-dioxo 2,3,4,5-tétrahydro 1H-1,5-benzodiazépine, leur procédé et les intermédiaires de préparation, leur application comme médicaments et les compositions les renfermant.**

(57) L'invention a pour objet les composés de formule (I) :

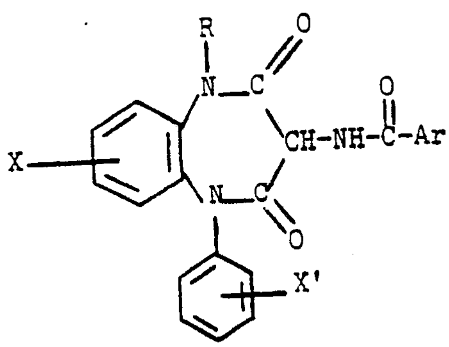

(I)

dans lesquels :

- X et X' identiques ou différents représentent un atome d'hydrogène, un atome d'halogène, un radical cyano, $NO_2$ ou $CF_3$ ;
- R représente un atome d'hydrogène ou un radical alkyle et ;
- Ar représente un radical aryle, un radical hétérocyclique aromatique ou un radical hétérocyclique fusionné avec un radical aryle.

Les composés de formule (I) présentent d'intéressantes propriétés pharmacologiques qui justifient leur utilisation en thérapeutique.

EP 0 376 849 A1

# Nouveaux dérivés de la 2,4-dioxo 2,3,4,5-tétrahydro 1H-1,5-benzodiazépine, leur procédé et les intermédiaires de préparation, leur application comme médicaments et les compositions les renfermant.

La présente invention concerne de nouveaux dérivés de la 2,4-dioxo 5-phényl 2,3,4,5-tétrahydro 1H-1,5-benzodiazépine, leur procédé et les intermédiaires de préparation leur application comme médicaments, et les compositions les renfermant.

L'invention a pour objet les composés de formule (I) :

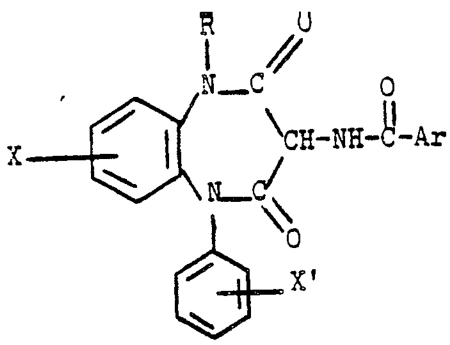

(I)

dans lesquels :

- X et X' identiques ou différents représentent un atome d'hydrogène, un atome d'halogène, un radical cyano, un radical $NO_2$, un radical $CF_3$, un radical alkyle ou alkoxy renfermant jusqu'à 8 atomes de carbone ;
- R représente un atome d'hydrogène ou un radical alkyle renfermant jusqu'à 8 atomes de carbone et ;
- Ar représente un radical aryle renfermant jusqu'à 14 atomes de carbone éventuellement substitué, un radical hétérocyclique aromatique éventuellement substitué ou un radical hétérocyclique fusionné avec un radical aryle éventuellement substitué, sous toutes les formes isomères possibles ainsi que leurs mélanges.

Les substituants, X et X' peuvent être en position quelconque sur les noyaux phényles. Lorsque X ou X' représente un atome d'halogène, il s'agit de préférence d'un atome de chlore ou de brome.

Lorsque X ou X' représente un radical alkyle ou alkoxy il s'agit de préférence d'un radical méthyle, éthyle, méthoxy ou éthoxy.

Lorsque R représente un radical alkyle, il s'agit de préférence d'un radical méthyle, éthyle, n-propyle, isopropyle ou n-butyle.

Lorsque Ar représente un radical aryle, il s'agit de préférence du radical phényle éventuellement substitué par un ou plusieurs substituants choisis dans le groupe des atomes d'halogène comme par exemple les atomes de chlore ou de brome, les radicaux alkoxy renfermant jusqu'à 4 atomes de carbone comme par exemple le radical méthoxy ou éthoxy, les radicaux alkyle renfermant jusqu'à 8 atomes de carbone comme par exemple le radical méthyle ou éthyle.

Lorsque Ar représente un radical hétérocyclique aromatique, il s'agit de préférence des radicaux pyridinyle, thiophényle, oxazolyle ou isoxazolyle.

Lorsque Ar représente un radical hétérocyclique fusionné avec un noyau phényle, il s'agit de préférence d'un noyau indolyle, benzofuranyle ou quinolinyle.

L'invention a plus particulièrement pour objet les composés de formule (I) dans lesquels R représente un radical alkyle renfermant jusqu'à 4 atomes de carbone comme par exemple un radical méthyle, ceux dans lesquels X' représente un atome d'hydrogène, ceux dans lesquels X représente un atome d'hydrogène, ou un atome d'halogène comme par exemple un atome de chlore en position 7.

L'invention a plus particulièrement pour objet les composés dont la préparation est donnée ci-après dans la partie expérimentale et tout spécialement les composés des exemples 1 et 2.

Les composés de formule (I) sont des agonistes ou des antagonistes de la cholecystokinine dont des sites de liaison ont été mis en évidence aux niveaux central et périphérique. La cholecystokinine est un peptide largement distribué dans le cerveau, notamment dans le cortex, le striatum, l'hippocampe, le tegmentum ventral, le septum et l'hypothalamus.

La cholecystokinine est également secrétée au niveau périphérique par l'intestin grêle, son action se manifeste notamment par la stimulation de la contraction vésiculaire, une augmentation de la secrétion biliaire, le contrôle de la secrétion enzymatique pancréatique, une action sur les contractions gastriques, une action sur la motilité intestinale. Elle pourrait agir dans certains cas sur la pression artérielle et

influencer les systèmes immunitaires.

La cholecystokinine coexiste dans certains neurones centraux avec la dopamine. Elle intervient également dans des mécanismes impliquant l'acéthylcholine, le gaba, la sérotonine, les opioïdes, la somatostatine, la substance P et des canaux ioniques.

Son administration provoque des modifications physiologiques : ptose palpébrale, hypothermie, hyperglycémie, catalepsie, et comportementales : hypolocomotricité, diminution de l'exploration, analgésie, action dans les apprentissages, modification du comportement sexuel et satiété.

Selon les doses, elle se comporte comme un agoniste ou un antagoniste dopaminergique.

Les composés de formule (I) peuvent donc être utilisés comme médicaments dans le traitement de certains troubles du comportement alimentaire, de l'obésité, dans des troubles des comportements, émotionnel, sexuel et mnésique, dans la schizophrénie et dans divers troubles de la sphère gastro-intestinale.

L'invention a donc pour objet à titre de médicaments les composés de formule (I), et tout particulièrement les composés dont la préparation est donnée ci-après dans la partie expérimentale, notamment les produits des exemples 1 et 2.

La posologie, variable selon le produit utilisé et l'affection en cause peut s'échelonner, par exemple, entre 0,05 mg et 100 mg par jour chez l'adulte par voie orale.

La présente demande a encore pour objet les compositions pharmaceutiques qui contiennent, à titre de principe actif, au moins un des médicaments précités. Ces compositions sont réalisées de façon à pouvoir être administrées par la voie digestive ou parentérale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation caractérisé en ce que l'on soumet un composé de formule II :

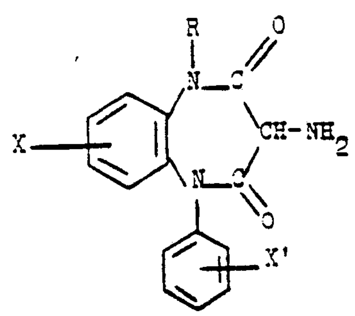

(II)

dans laquelle R, X et X′ conservent leur signification précédente à l'action d'un acide ou un dérivé d'acide de formule (III) :

$Ar\overset{O}{\overset{||}{C}}-OH$ (III)

dans laquelle Ar conserve sa signification précédente, pour obtenir le composé de formule (I) correspondant.

Dans un mode de réalisation préféré du procédé de l'invention, le composé (III) est utilisé sous forme d'acide, de chlorure d'acide ou d'anhydride d'acide.

Les composés de formule (II) utilisés comme produits de départ du procédé de l'invention sont des produits nouveaux et sont en eux-mêmes un objet de la présente invention.

L'invention a tout particulièrement pour objet les composés de formule (II) dont la préparation est donnée ci-après dans la partie expérimentale.

Les composés de formule (II) peuvent être préparés selon un procédé caractérisé en ce que l'on soumet un composé de formule (IV) :

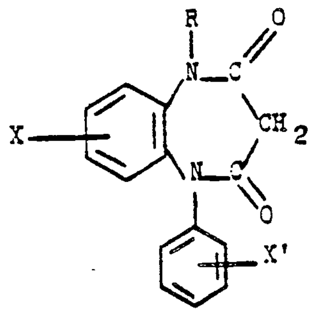

(IV)

à l'action d'un agent capable d'introduire un radical =NOH, par exemple le nitrile d'amyle, d'isoamyle, d'isopentyle ou de terbutyle pour obtenir le composé de formule (V) :

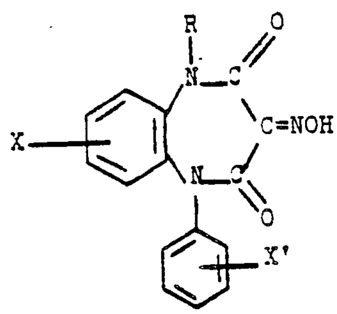

(V)

que l'on soumet à l'action d'un agent de réduction, par exemple l'hydrogène en présence d'un catalyseur comme le nickel de Ramey, le palladium sur charbon, ou encore d'un autre agent réducteur par exemple Li $AlH_4$ ou encore le zinc dans l'acide acétique ou le sodium dans l'éthanol pour obtenir le composé de formule (II) correspondant :

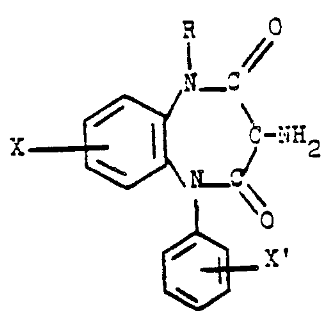

(II)

Lorsque l'on veut préparer les composés de formule (II) dédoublés, on peut utiliser une variante du procédé précédent, caractérisé en ce que l'on soumet un composé de formule (IV) :

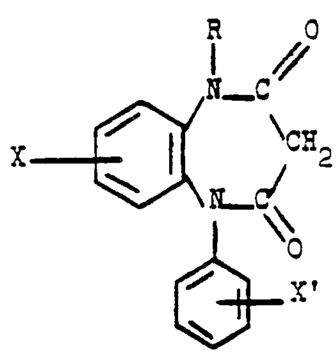

(IV)

à l'action d'un agent d'halogénation, pour obtenir le composé de formule (V') :

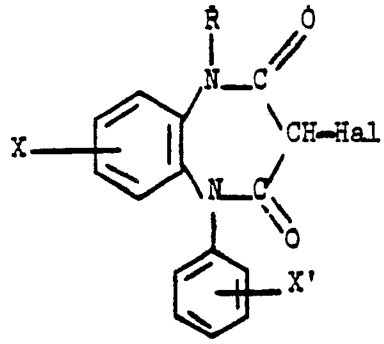

(V')

que l'on soumet à l'action d'un composé de formule :

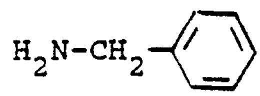

pour obtenir le composé de formule (VI') :

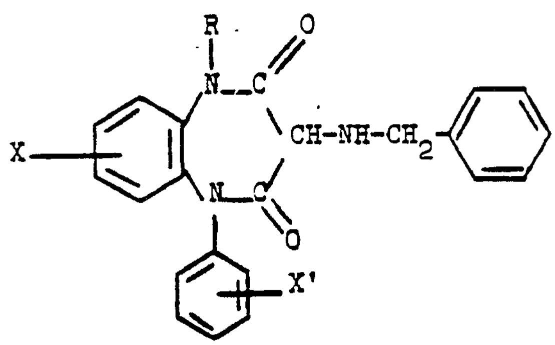

(VI')

que l'on soumet à l'action d'un agent de réduction pour obtenir le composé de formule (II) :

(II)

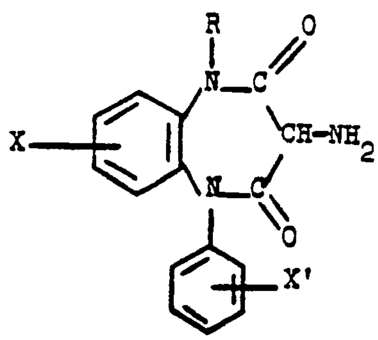

R,S

que l'on soumet à l'action d'un composé de formule :

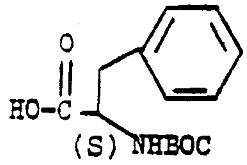

pour obtenir le composé de formule :

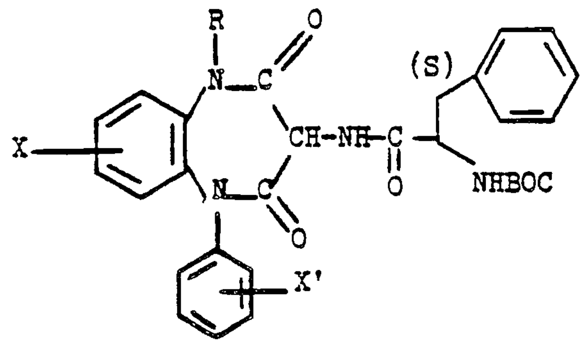

que l'on soumet à l'action d'un acide comme l'acide chlorhydrique ou l'acide trifluoroacétique pour obtenir le composé de formule :

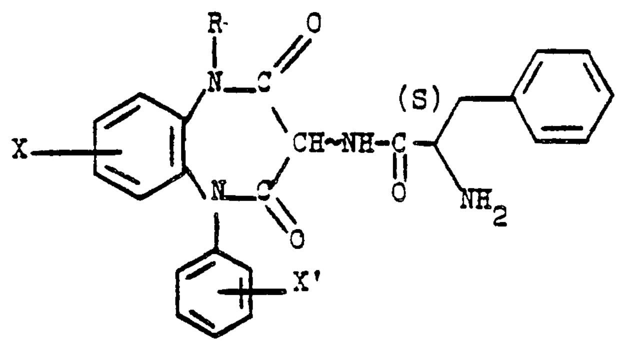

On sépare les 2 diastéréoisomères et chacun des deux composés obtenus est soumis à la dégradation d'Edman pour donner les deux énantiomères du composé de formule (II).

**Exemple 1 : N-(7-chloro 2,4-dioxo 1-méthyl 5-phényl 2,3,4,5-tétrahydro 1H-1,5-benzodiazepin-3-yl) 1H-indole-2-carboxamide.**

On met sous agitation à température ambiante pendant 18 heures un mélange constitué de 1,58 g (5 mmoles) de 3-amino 2,4-dioxo 7-chloro 1-méthyl 5-phényl 2,3,4,5-tétrahydro 1H-1,5-benzodiazépine, 0,89 g d'acide 2-indol carboxylique, 1,05 g de 1-éthyl 3(3-diméthylamino) propyl carbodiimide, 1,02 g de 1-hydroxy benzotriazole, 50 ml de dichlorométhane. On essore le précipité formé et le lave avec du dichlorométhane. On obtient 1,36 g de produit attendu fondant vers 280°.
Rdt 59 %.

| Microanalyse : | | | | |
|---|---|---|---|---|
| Calculé %<br>Trouvé | C = 65,43<br>65,42 | H = 4,17<br>4,0 | N = 12,21<br>12,3 | Cl = 7,72<br>8,1 |

En opérant comme à l'exemple 1, en suivant le schéma réactionnel :

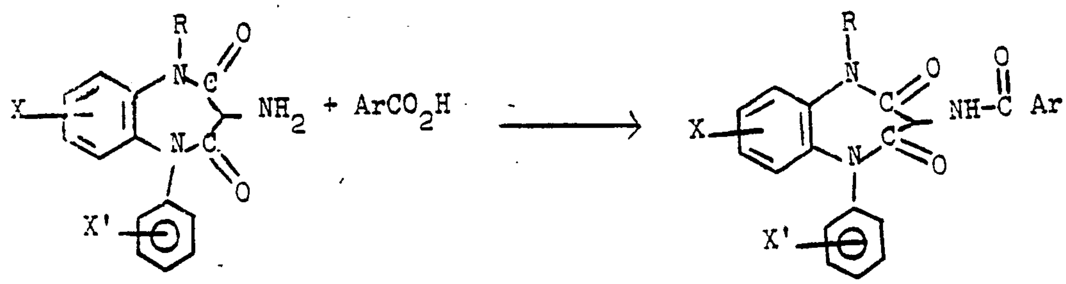

on a obtenu les produits suivants :

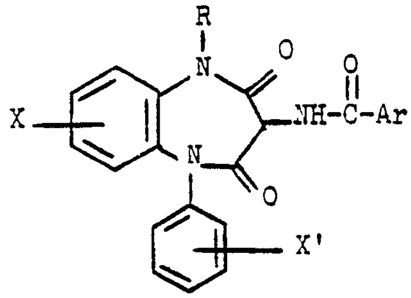

| Ex. | R | X | X' | Ar | Stéréo-chimie | Constante physique |
|---|---|---|---|---|---|---|
| 2 | $CH_3$ | H | H | 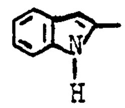 | R ou S | F ~ 170°C |
| 3 | $CH_3$ | H | H | 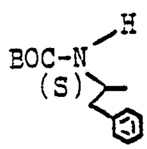 | RS | ccm rf = 0,35 support $SiO_2$ éluant $CHCl_2$ 95 MeOH 5 |
| 4 | $CH_3$ | H | H | 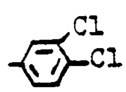 | RS | F = 215°C |
| 5 | $CH_3$ | H | H | 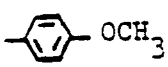 | RS | F = 234°C |
| 6 | $CH_3$ | H | H | 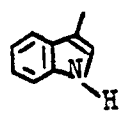 | RS | F > 280°C |

**Preparation 1 : 3-amino-7-chloro-1-méthyl-5-phényl-1H-1,5-benzodiazépine-2,4(3H,5H)-dione.**

__Stade A :__ 3-oxime de 7-chloro-1-méthyl-5-phényl-1H-1,5-benzodiazépine-2,3,4(5H)-trione.

On met en suspension 3 g de 7-chloro-1-méthyl-5-phényl-1H-1,5-benzodiazépine-2,4(3H,5H)-dione dans 30 ml de terbutanol. On ajoute par petites fractions 1,12 g de terbutylate de potassium. En maintenant la température à 20° par un bain d'eau, on ajoute 2,3 ml de nitrite de ter-amyle. La solution s'épaissit et un précipité se forme. On laisse 16 heures à la température ambiante ; on essore le précipité formé (sel de potassium de l'oxime), on le dissout dans 20 ml d'eau et on acidifie par de l'acide acétique. On extrait le

précipité formé. On lave la phase organique à l'eau. On la sèche et on concentre. On récupère 2,8 g de produit attendu.
Rdt = 85 % - F≈50°.

| Microanalyse : | | | | |
|---|---|---|---|---|
| Calculé % | C = 58,28 | H = 3,67 | N = 12,74 | Cl = 10,75 |
| Trouvé | 58,1 | 3,9 | 12,8 | 10,6 |

<u>Stade B</u> : 3-amino-7-chloro-1-méthyl-5-phényl-1H-1,5-benzodiazépine-2,4(3H,5H)-dione.

On hydrogène 560 mg (1,7 mmole) d'oxime précédemment obtenue en présence de nickel de Raney (environ 500 mg) dans 30 ml d'éthanol sous une pression de 800 mbars pendant 16 heures.

On filtre le catalyseur, on concentre le solvant, on reprend dans 20 ml de $CH_2Cl_2$. On lave la phase organique à l'eau, la sèche sur $MgSO_4$ et on élimine le solvant. On récupère 400 mg de produit attendu sous forme de résine.
Rdt = 23 %.

| Microanalyse : | | | | |
|---|---|---|---|---|
| Calculé % | C = 60,86 | H = 4,47 | N = 13,31 | Cl = 11,23 |
| Trouvé | 61,12 | 4,5 | 12,9 | 11,2 |

**Préparation 2 : 3-amino-1-méthyl-5-phényl-1H-1,5-benzodiazépine 2,4(3H,5H)-dione.**

<u>Stade A</u> : 1-méthyl-5-phényl-1H-1,5-benzodiazépine-2,4(3H,5H)-dione.

On hydrogène pendant 24 heures sous une pression de 800 mbars, une suspension renfermant 103 g de clobazam, 1 litre de méthanol, 23 g de potasse en solution dans 400 $cm^3$ de méthanol et 50 g de nickel de Raney préalablement lavé à l'eau et au méthanol. On ajoute 1 litre de chlorure de méthylène, filtre le catalyseur, et concentre les solvants sous pression réduite. On reprend au chlorure de méthylène, lave à l'eau, sèche sur sulfate de magnésium et concentre sous pression réduite. On recristallise le produit obtenu dans le méthanol, le lave à l'éther, le sèche et obtient 76,69 g du produit recherché fondant à 173° C.

<u>Stade B</u> : 3-bromo-1-méthyl-5-phényl-1H-1,5-benzodiazépine-2,4-(3H,5H)-dione.

On irradie avec une lampe de 500 watts et introduit goutte à goutte vers 40° C une solution renfermant 5 $cm^3$ de brome et 30 ml de tétrachlorure de carbone dans une solution renfermant 25 g de produit préparé au stade A et 750 $cm^3$ de tétrachlorure de carbone. A la fin de l'introduction, on éteint la lampe, refroidit et ajoute 500 $cm^3$ de chlorure de méthylène. On lave la phase organique à l'aide d'une solution saturée de carbonate de soude, puis à l'eau. On chromatographie sur silice le produit obtenu en éluant à l'aide du mélange acétate d'éthyle-chlorure de méthylène 7-3. On obtient ainsi 10,5 g de produit recherché fondant à 132° C.

<u>Stade C</u> : 1-méthyl-5-phényl-3-<(phénylméthyl) amino>-1H-1,5-benzodiazépine-2,4(3H,5H)-dione.

On chauffe à 100° C pendant 3 heures, un mélange renfermant 50 mg du produit obtenu au stade B et 2 $cm^3$ de benzylamine. On verse le milieu réactionnel dans l'eau. On essore le produit obtenu. On le lave et

le sèche en présence d'anhydride phosphorique. On obtient 50 mg de produit recherché. RMN $CDCl_3$ ppm
H de $CH_2$ benzyle 3,86 ppm

H de $-\overset{O}{\overset{||}{C}}-CH-\overset{O}{\overset{||}{C}}$ 4,17 ppm

**Stade D** : 3-amino-1-méthyl-5-phényl-1H-1,5-benzodiazépine-2,4-(3H,5H)-dione.

On hydrogénolyse sous 1800 mb de pression 3,1 g (8,4 mmoles) de la benzylamine obtenue précédemment dans 100 $cm^3$ de méthanol en présence de 900 mg de charbon palladié à 10 %. Après absorption de la quantité d'hydrogène attendue on filtre le catalyseur et on concentre le méthanol. On obtient une poudre blanche qu'on recristallise dans l'éthanol. On récupère 1,67 g d'amine attendue. F = 186°.
Spectre IR : NH 3383-3321 $cm^{-1}$.

**Stade E** : (S)<2<(2,4-dioxo-1-méthyl-5-phényl 2,3,4,5-tétrahydro-1H-1,5- benzodiazépin-3-yl) amino>-2-oxo-1-(phénylméthyl) éthyl>-carbamate de 1,1- diméthyléthyle.

On agite pendant 16 heures un mélange composé de 2,4 g de produit obtenu au stade précédent, 40 ml de chlorure de méthylène, 2,26 g (8,5 mmoles) de BOC-L-phénylalanine, 1,8 g (9,4 mmoles) de dicyclohexylcarbodiimide et 1,26 g (8,5 mmoles) de 4-pyrrolidinopyridine. On verse ensuite dans 100 ml d'acide chlorhydrique, décante la phase organique et le lave trois fois à l'eau. On sèche et élimine le solvant. On obtient 5 g d'une mousse jaunâtre.
RMN
BOC 1,38 ppm (s)
ØCH2-CH 3,03 et 3,27 ppm
$N-CH_3$ 3,55 ppm (s).

**Stade F** : <(alphas)R*,S*> alpha-amino-N-(2,4-dioxo-1-méthyl-5-phényl-2,3,4,5-tétrahydro-1H-1,5-benzodiazépin-3-yl)-benzène-propanamide.

Isomère A et isomère <alphaS(R*,R*)> : isomère B.

On dissout 5 g du produit obtenu précédemment dans 80 $cm^3$ d'acétate d'éthyle et on fait passer, à 5°, un courant d'acide chlorhydrique sec pendant 20 minutes. On revient à température ambiante, on lave la solution organique par une solution saturée de bicarbonate de soude puis avec une solution saturée de chlorure de sodium. On sèche et élimine le solvant. On récupère environ 4,5 g d'une mousse jaune que l'on chromatographie sur silice en éluant par un mélange $CHCl_2$ : 89 MeOH : 10 AcOH : 1.

On isole
le premier isomère A P = 1,78 g Rf = 0,52 ;
le deuxième isomère B P = 1,68 g Rf = 0,48.

**Stade G** : 3-amino-7-chloro-1-méthyl 5-phényl-1H-1,5-benzodiazépine-2,4(3H,5H)-dione.

On agite pendant une heure à la température ambiante, un mélange renfermant 0,238 g de l'isomère A obtenu au stade précédent, 4 $cm^3$ de chlorure de méthylène, et 0,125 $cm^3$ d'isothiocyanate de phényle. On concentre le solvant. On ajoute 3 $cm^3$ d'acide trifluoroacétique, on agite une heure à température ambiante et concentre sous pression réduite. On reprend dans un mélange chlorure de méthylène-méthanol (1/1) pour dissoudre la gomme formée et on verse sur 5 ml de d'ammoniaque concentré. On agite une nuit à température ambiante. On extrait au chlorure de méthylène, lave à l'eau, on sèche et concentre. On obtient 40 mg d'une résine blanche correspondant à l'amine optiquement active. Rdt = 25 %.
CCM RF = 0,23 AcOEt/$NEt_3$ 97/3.

De la même manière en partant de l'isomère B obtenu précédemment on obtient le deuxième antipode.

Formes pharmaceutiques

Exemple 7 : Comprimés

On a préparé des comprimés répondant à la formule suivante :

| | |
|---|---|
| - Produit de l'exemple 3 | 20 mg |
| - Excipient q.s. pour un comprimé terminé à | 300 mg |

(Détail de l'excipient : lactose, amidon de blé, amidon traité, amidon de riz, stéarate de magnésium, talc).

Exemple 8 : Gélules

On a préparé des gélules répondant à la formule suivante :

| | |
|---|---|
| - Produit de l'exemple 2 | 50 mg |
| - Excipient q.s. pour une gélule terminée à | 300 mg |

Détail de l'excipient : talc, stéarate de magnésium, aérosil).

## Etude biologique

1) Récepteurs centraux

On prélève les cortex de 20 rats mâles pesant de 150 à 200 g, ces cortex sont broyés au Polytron dans du sucrose 0,32 M.

Après centrifugation le surnageant est recueilli et centrifugé.

Les culots sont remis en suspension dans 120 ml tampon HEPES pH 7,4 (Hepes 10 mM, NaCl 130 mM, $MgCl_2$, $6H_2O$ 5 mM, bacitracine 250 mg/l, PMSF 1 mg/l), et recentrifugés.

Les culots sont repris dans 120 ml de tampon Hepes pH 7,4 et recentrifugés à 30000 g 30 mn.

Les culots ainsi obtenus sont repris par 500 ml de tampon Hepes pH 7,4, ce qui permet d'obtenir 240 aliquotes de 2 ml d'homogénat.

L'indication est faite à 25° C 30 mn en présence de 0,5 nM de 3H CCK8 et du produit à tester (10000 $\mu$M pour 1 dose, ou avec une gamme de 7 doses ou de CCK8 froide ($10^{-6}$M , qui est le produit de référence.

Après retour à 0° C des aliquotes d'homogénat, ceux-ci sont filtrés sur filtres Whatman GF/B et les filtres lavés par 3x5 ml d'un tampon Tris HCl 50 mM pH 7,4.

Les résultats sont exprimés en $CI_{50}$ : concentration nécessaire pour inhiber de 50 % la radioactivité spécifique fixée.

2) Récepteurs périphériques

Les pancréas de 3 rats mâles, 150-200 g, sont prélevés et broyés au Polytron (4 broyages, vitesse 3, avec un intervalle de 10 secondes entre les broyages) ; l'homogénat est filtré sur une gaze, puis centrifugé à 30000 g 30 minutes.

Les culots obtenus sont repris par 400 volumes ( 600 ml) de tampon Tris 50 mM HCl pH 7,4, contenant BSA 2 g/l, bacitracine 0,1 mM, $MgCl_2$ 5 mM, dithiothreitol 5 mM.

Des aliquotes d'homogénat de 2 ml sont incubés à 25° C 60 mn, en présence de 0,2 nM 3H CCK8 et du produit à tester (10000 $\mu$M pour 1 dose, ou avec 1 gamme de 7 doses) et de CCK8 froide ($10^{-6}$ M) qui est le produit de référence.

Après retour à 0° C, les aliquotes sont filtrés sur filtres Whatman GF/B, prélavés dans une solution de polyéthylène imine à 0,05 %, lavés par 3x5 ml de tampon Tris HCl 50 mM pH 7,4.

Les résultats sont exprimés en $CI_{50}$ : concentration nécessaire, pour inhiber de 50 % la radioactivité spécifique fixée. $CI_{50}$ n mol

| Exemple | CCK Périphérique | CCK Central |
|---|---|---|
| 1 | 20 | 2200 |
| 2 | 14 | > 10000 |

**ACTION SUR LA PRISE ALIMENTAIRE CHEZ LE RAT**

Technique :

Les essais sont réalisés sur des lots de 5 rats de 250 ± 10 g dans les conditions suivantes : les animaux sont placés individuellement dans des cages ayant une mangeoire décrite par FREGLY (J.Appl.Physiol., 1960, 15, 539) qui présente l'avantage d'éviter le gaspillage de nourriture constituée par de la provende pulvérisée. Les rats sont habitués à prendre leur ration quotidienne en 5 h consécutives, l'eau de boisson étant offerte ad libitum dans des biberons de verre.

Les quantités de nourriture ingérées sont déterminées individuellement par la pesée des mangeoires. Les consommations sont suivies d'heure en heure 40 pendant 5 heures après l'administration de 10 mg/kg i.p. du composé. Les quantités consommées sont exprimées en g/100 g de poids corporel, par heure.

Les moyennes sont comparées à celles obtenues chez animaux témoins par un test de DUNNETT.

Résultats :

A la dose de 10 mg/kg par voie intrapéritonéale, le produit de l'exemple 1 manifeste une activité anorexigène.

Il réduit significativement de plus de 50 % la consommation alimentaire des animaux traités par rapport à celle des animaux témoins.

**Action sur l'iléon isolé de cobaye**

Méthode :

L'essai est réalisé sur des fragments d'iléon de cobaye mâles, placés sous une tension de 1 g dans une solution de Krebs aérée au carbogène et maintenue à 37° C. Les contractions sont enregistrées à l'aide d'un micrody- namomètre relié à un polygraphe.

L'iléon est laissé au repos pendant 30 minutes, puis la $CCK_8$ est ajoutée dans le bain à la concentration de $1.10^{-8}$M. On rince. Le produit à étudier est ajouté dans le bain, laissé en contact 1 minute avec l'organe, puis du $CCK_8$ ($1.10^{-8}$ M) est ajoutée dans le bain. L'antagonisme éventuel est exprimé en comparant les contractions engendrées par la $CCK_8$ avant et après contact du produit à tester.

Le produit de l'exemple 2 à une dose de $10^{-7}$ M présente une activité antagoniste intéressante.

**Revendications**

1) Les composés de formule (I) :

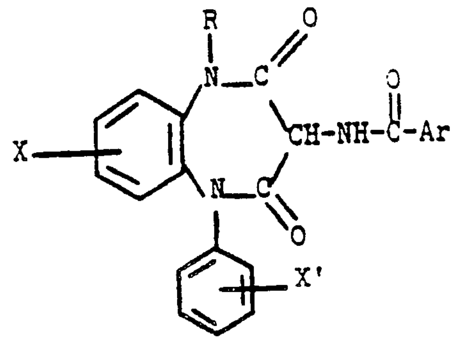

(I)

dans lesquels :

- X et X′ identiques ou différents représentent un atome d'hydrogène, un atome d'halogène, un radical cyano, un radical $NO_2$, un radical $CF_3$, un radical alkyle ou alkoxy renfermant jusqu'à 8 atomes de carbone ;
- R représente un atome d'hydrogène ou un radical alkyle renfermant jusqu'à 8 atomes de carbone et ;
- Ar représente un radical aryle renfermant jusqu'à 14 atomes de carbone éventuellement substitué, un radical hétérocyclique aromatique éventuellement substitué ou un radical hétérocyclique fusionné avec un radical aryle éventuellement substitué, sous toutes les formes isomères possibles ainsi que leurs mélanges.

2) Les composés de formule (I) tels que définis à la revendication 1 dans lesquels R représente un radical alkyle renfermant jusqu'à 4 atomes de carbone.

3) Les composés de formule (I) tels que définis à la revendication 2 dans lesquels R représente un radical méthyle.

4) Les composés de formule (I) tels que définis à l'une quelconque des revendications 1 à 3 dans lesquels X′ représente un atome d'hydrogène.

5) Les composés de formule (I) tels que définis à l'une quelconque des revendications 1 à 4 dans lesquels X représente un atome d'hydrogène.

6) Les composés de formule (I) tels que définis à l'une quelconque des revendications 1 à 4 dans lesquels X représente un atome d'halogène.

7) Les composés de formule (I) tels que définis à la revendication 6 dans lesquels X représente un atome de chlore en position 7.

8) Les composés de formule (I) tels que définis à la revendication 1 dont les noms suivent :

N-(7-chloro 2,4-dioxo-1-méthyl-5-phényl-2,3,4,5-tétrahydro-1H-1,5- benzodiazepin-3-yl)-1H-indole-2-carboxamide.

N-<2,4-dioxo-1-méthyl-5-phényl-2,3,4,5-tétrahydro-1H-<1,5>-benzodiazepin-3- yl>-1H-indole-2-carboxamide.

9) A titre de médicaments, les composés de formule (I) tels que définis à l'une quelconque des revendications 1 à 7.

10) A titre de médicaments, les composés de formule (I) tels que définis à la revendication 8.

11) Les compositions pharmaceutiques renfermant comme principe actif au moins un médicament défini à la revendication 9 ou 10.

12) Procédé de préparation des composés de formule (I) tels que définis à l'une quelconque des revendications 1 à 8 caractérisé en ce que l'on soumet un composé de formule (II) :

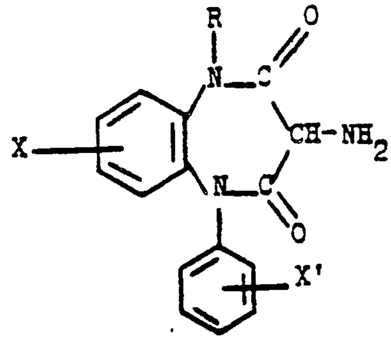

(II)

dans laquelle R, X et X′ conservent leur signification précédente à l'action d'un acide ou un dérivé d'acide

de formule (III) :

$$Ar\overset{O}{\overset{\|}{C}}-OH \quad (III)$$

dans laquelle Ar conserve sa signification précédente, pour obtenir le composé de formule (I) correspondant.

13) A titre de produits chimiques nouveaux, les composés de formule (II) :

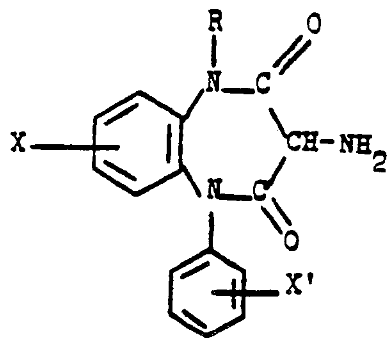

(II)

tels que définis à la revendication 12.

14) A titre de produits chimiques nouveaux tels que définis à la revendication 13 ;
- le 3-amino-7-chloro-1-méthyl-5-phényl-1H-1,5-benzodiazépine 2,4(3H,5H)- dione ;
- le 3-amino-1-méthyl-5-phényl-1H-1,5-benzodiazépine 2,4(3H,5H) dione.

Office européen des brevets

# RAPPORT DE RECHERCHE EUROPEENNE

Numero de la demande

EP 89 40 3668

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | JUSTUS LIEBIGS ANNALEN DER CHEMIE, vol. 763, 24 novembre 1972, pages 66-74, Weinheim, DE; K.-H. WEBER et al.: "Reaktionen von N.N'-disubstituierten 1.5-Benzodiazepin-2.4-dionen in 3-Stellung"<br>* En entier, en particulier page 69, composé 3 *<br>--- | 1-14 | C 07 D 243/12<br>C 07 D 403/12<br>C 07 K 5/06<br>A 61 K 31/55<br>A 61 K 37/02 |
| Y | JOURNAL OF ORGANIC CHEMISTRY, vol. 52, no. 5, 6 mars 1987, pages 955-957, American Chemical Society, Easton, US; P.J. REIDER et al.: "Crystallization-induced asymmetric transformation: stereospecific synthesis of a potent peripheral CCK antagonist"<br>* En entier *<br>----- | 1-12 | |

| DOMAINES TECHNIQUES RECHERCHES (Int. Cl.5) |
|---|
| C 07 D 243/00<br>C 07 D 403/00<br>C 07 K 5/00 |

Le présent rapport a été établi pour toutes les revendications

| Lieu de la recherche | Date d'achèvement de la recherche | Examinateur |
|---|---|---|
| LA HAYE | 02-04-1990 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................................
& : membre de la même famille, document correspondant

EPO FORM 1503 03.82 (P0402)